(19)

Europäisches
Patentamt
European
Patent Office
Office européen
des brevets

(11)  **EP 4 076 194 B1**

(12)  **EUROPEAN PATENT SPECIFICATION**

(45) Date of publication and mention
of the grant of the patent:
**06.03.2024  Bulletin 2024/10**

(21) Application number: **20845630.1**

(22) Date of filing: **17.12.2020**

(51) International Patent Classification (IPC):
*A61B 5/397* (2021.01)   *A61B 5/395* (2021.01)

(52) Cooperative Patent Classification (CPC):
**A61B 5/397; A61B 5/395**

(86) International application number:
**PCT/SI2020/050028**

(87) International publication number:
**WO 2021/126093 (24.06.2021 Gazette 2021/25)**

(54) **A PROCESS AND A DEVICE FOR DECOMPOSITION OF COMPOUND MUSCLE ACTION POTENTIALS**

VERFAHREN UND VORRICHTUNG ZUR ZERLEGUNG VON ZUSAMMENGESETZTEN MUSKELWIRKUNGSPOTENZIALEN

PROCÉDÉ ET DISPOSITIF DE DÉCOMPOSITION DE POTENTIELS D'ACTION MUSCULAIRE COMPOSITES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.12.2019  SI 201900252**

(43) Date of publication of application:
**26.10.2022  Bulletin 2022/43**

(73) Proprietor: **Univerza v Mariboru**
**2000 Maribor (SI)**

(72) Inventor: **HOLOBAR, Ales**
**3214 Zrece (SI)**

(74) Representative: **Patentni Biro AF d.o.o.**
**Kotnikova 32, p.p. 2706**
**1001 Ljubljana (SI)**

(56) References cited:
- **PENG YUN ET AL: "Motor unit number estimation based on high-density surface electromyography decomposition", CLINICAL NEUROPHYSIOLOGY, ELSEVIER SCIENCE, IE, vol. 127, no. 9, 25 June 2016 (2016-06-25) , pages 3059-3065, XP029675533, ISSN: 1388-2457, DOI: 10.1016/J.CLINPH.2016.06.014**
- **MARCO GAZZONI ET AL: "A new method for the extraction and classification of single motor unit action potentials from surface EMG signals", JOURNAL OF NEUROSCIENCE METHODS, vol. 136, no. 2, 1 July 2004 (2004-07-01), pages 165-177, XP055186580, ISSN: 0165-0270, DOI: 10.1016/j.jneumeth.2004.01.002**
- **NING YONG ET AL: "Surface EMG Decomposition Based on K-means Clustering and Convolution Kernel Compensation", IEEE JOURNAL OF BIOMEDICAL AND HEALTH INFORMATICS, IEEE, PISCATAWAY, NJ, USA, vol. 19, no. 2, 1 March 2015 (2015-03-01), pages 471-477, XP011574273, ISSN: 2168-2194, DOI: 10.1109/JBHI.2014.2328497 [retrieved on 2015-03-03]**
- **HOLOBAR A ET AL: "Multichannel Blind Source Separation Using Convolution Kernel Compensation", IEEE TRANSACTIONS ON SIGNAL PROCESSING, IEEE SERVICE CENTER, NEW YORK, NY, US, vol. 55, no. 9, 1 September 2007 (2007-09-01), pages 4487-4496, XP011190585, ISSN: 1053-587X, DOI: 10.1109/TSP.2007.896108**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field of the invention

The invention relates to a method and device for decomposition of compound muscle action potentials, CMAPs, into contributions of individual motor units. The invention relates to stimulated muscle contractions. Muscle contractions can be stimulated by direct (e.g. electrical or mechanical muscle stimulation) or indirect (e.g. magnetic stimulation of the motor cortex) stimulation. The invention allows an analysis of several different compound muscle potentials such as H-reflexes, F waves, M waves and motor evoked potentials.

The technical problem

Skeletal muscles spatially distribute and strengthen the neural codes that control human movement and have been the subject of intensive research in neurophysiology, neurology, rehabilitation, prosthetics, ergonomics, and many other sciences. Each motor nerve innervates from a few tens to a few hundred muscle fibres and forms with them the basic functional unit of movement, the so-called motor unit. Information on the activity of motor units has contributed to a better understanding of pathologies such as stroke and pathological tremor and to neurophysiological studies of reflexes and aging. In many different healthy and pathological conditions, neuromuscular junctions remain stable, so the functioning of individual motor units authentically reflects the codes of motor neurons. These codes can be detected by decomposing needle or surface electromyograms, i.e. EMG.

Compound muscle action potential i.e. CMAP is the sum of the motor unit action potentials, i.e. MUAPs in a skeletal muscle stimulated by electrical, magnetic, or mechanical stimulation of nerves or muscles, or transcranial electrical i.e. TES or magnetic stimulation i.e. TMS of the motor cortex. In the latter case, the CMAP is called motor evoked potential i.e. MEP.

The CMAP analysis is routinely used in clinical and neurophysiological studies for non-invasive in vivo assessment of the functional state of the human motor system, for assessing the integrity of the motor tracts, and for determining responses of the neuromuscular system to training, rehabilitation, and degeneration due to various neuromuscular diseases, in studies of corticospinal excitability and conductivity of motor nerves, and in the analysis of fatigue and biomechanical responses of skeletal muscles.

In a typical supramaximal stimulation, the stimulatory current or voltage is gradually increased until their increase reaches the maximum amplitude of the CMAP. This leads to reproducible CMAP scans, since at that time CMAP consists of the MUAPs of all the motor units in the stimulated muscle tissue. When measurements are performed on the skin surface, MUAPs travel through a subcutaneous tissue located between the motor unit and the measuring electrode, and their shape reflects muscle architecture and geometry, distribution of motor units in the muscle tissue, electrical properties of the subcutaneous tissue, and properties of the system for capturing EMG signals.

In submaximal stimulations, elicited CMAPs are highly variable. This variability in the CMAP forms is mainly due to incomplete muscle stimulation or neighbouring muscle contributions (i.e. muscle cross-talk), especially in areas with high muscle spatial density, such as the forearm. The problem of variability of submaximal CMAPs has not yet been systematically investigated, mainly due to the lack of techniques for the decomposition of CMAPs. A distal supramaximal electrical stimulus propagating in the antidromic direction is known to elicit an electrical response, i.e. an F wave in about 2 to 5% of the total motor nerve population. In addition, the motor neurons involved in generating the F wave differ from the motor neurons activated at low to moderate rates of voluntary muscle contractions. Similarly, the order of recruitment of motor units in submaximal orthodromic stimulations that generate M waves differs from that in voluntary muscle contractions. In general, the order of recruitment of motor units in stimulations is usually reversed relative to the order of recruitment in voluntary contractions. However, in practice, this order depends on the relative location of the nerve with respect to the stimulation electrodes and on many other stimulation parameters, so it needs to be checked again and again in experimental measurements. These facts prove the existence of a need for reliable methods for direct assessment of the activity of individual motor neurons from compound muscle potentials.

The technical problem solved by the invention is how to assess the time moments in which the motor unit action potentials, i.e. MUAPs, appear as the responses of motor units to external stimulation. In contrast to voluntary muscle contractions, electrical responses of motor units to external stimuli are more or less concurrent and the MUAPs in the CMAP overlap at least in part. This concurrence of motor unit responses has so far prevented a detailed analysis of individual MUAPs and, consequently, the decomposition of CMAPs into individual motor unit contributions. Therefore, these methodological limitations prevented also a more detailed analysis of individual motor unit responses to various external excitations, and a more detailed analysis of the excitability of the motor cortex or motor nerves.

Motor neurons and associated muscle fibres conduct electrical stimuli at different speeds. This results in a time scattering of individual motor unit contributions in the CMAP, regardless of the intensity of the stimulation. With muscle fatigue, changes in the rate of MUAP conduction occur, which differ significantly in different types of muscle fibres. This causes time changes in the MUAP distributions in the CMAP and consequently changes in the shape of the CMAP. By understanding this time scattering of MUAPs and by decomposing a CMAP into the contributions of slow and fast muscle fibres, skeletal muscle composition could be inferred in a completely non-invasive way. This information would be extremely important for understanding muscle aging, for better management of rehabilitation and training of athletes, and for early detection and objective monitoring of neuromuscular diseases, such as atrophies, that selectively affect a particular type of muscle fibres.

Similarly, the decomposition of a CMAP to the contributions of individual motor units could be used to improve the accuracy of the non-invasive assessment - well established in literature - of the number of motor axons that innervate a muscle. This would greatly improve the ability of objective and long-term monitoring of the myopathies and neuropathies.

State of the art

Several CMAP analysis techniques have been developed in the past. A search in the Pubmed database returns about 1,600 papers dealing with this topic. Among them, about 250 papers describe the use of the MUNE (Motor Unit Number Estimation) method or its variants to estimate the number of motor units based on a CMAP analysis. In the conventional MUNE method, the amplitude of the CMAP is normalized by the average amplitude of the MUAP, which is estimated by a relatively small number of motor units identified from bipolar EMG images of low to moderate voluntary contraction rates or by incrementally increasing an electrical stimulus. This methodology has several limitations. First, it largely ignores the negative destructive sums of MUAPs (i.e. mutual cancellation of MUAPs) in CMAPs. Second, at high muscle contractions, dozens of motor units are active at the same time, and their MUAPs overlap in time to form highly interfering EMG patterns. A reliable assessment of MUAPs at high contraction rates is therefore a difficult task that is usually skipped in the conventional MUNE methodology. It is important that in simulated conditions the order of recruitment of motor units is usually reversed, which means that the motor units recruited at high voluntary contractions are very likely to be active even at relatively low stimulation. The incremental increase in electrical stimuli has similar disadvantages, since without CMAP decomposition, it is extremely difficult to infer the actual number of newly excited motor units in a single increment. This leads to rather inaccurate estimates of the number of motor units in the muscle under study.

An accurate analysis of MUAPs at high rates of muscle contraction has only become possible with the recent development of EMG surface signal decomposition techniques recorded during voluntary muscle contractions, where the motor unit action potentials are asynchronous. The University of Maribor developed these techniques in 2004, i.e. Convolution Kernel Compensation - CKC method, and has been one of the leading players in the field of EMG surface signal analysis ever since. The CKC method for decomposing EMG signals recorded during voluntary muscle contractions has been successfully tested in more than 15 different skeletal muscles and has identified up to 50 active motor units in a single voluntary muscle contraction. It has also been used successfully at very high contraction rates (>70% of maximum voluntary contraction), demonstrating the ability to accurately identify motor units with a high recruitment threshold. Equally important, the method has already been tested in the analysis of pathological tremor, indicating its robustness towards high levels of motor unit synchronisation. Such an advanced decomposition methodology supports the analysis of a large number of motor units and can be used compared to other methods to identify a more representative set of MUAPs that make up a CMAP. However, the CKC method is unsuitable for a CMAP analysis because the synchronisation level of the MUAPs that make up the CMAP exceeds the synchronisation levels of the MUAPs supported by the CKC method.

Interestingly, the use of a multichannel CMAP analysis is declared in only 31 of the 1,600 papers mentioned above, although the benefits of interpreting multichannel EMG signals have been clearly demonstrated in voluntary muscle contractions. In all other studies, conventional bipolar EMG measurements were used to measure muscle responses. In contrast to the high spatial density of multichannel EMG signals, bipolar EMG measurements do not support a detailed spatial analysis of a CMAP and its decomposition into individual MUAPs. This reduces the accuracy of CMAP analyses and the neuromuscular system estimates obtained from them. Namely, multi-channel measurements of motor units enable a view of motor units from several perspectives and significantly increase the ability to distinguish their contributions in EMG surface signals.

Peng et al. (2016, DOI: 10.1016/j.clinph.2016.06.014) disclose a motor unit number estimation (MUNE) technique using high density surface electromyography (EMG) decomposition. The K-means clustering convolution kernel compensation algorithm was employed to detect the single motor unit potentials (SMUPs) from high-density surface EMG recordings of the biceps brachii muscles in eight healthy subjects. Contraction forces were controlled at 10%, 20% and 30% of the maximal voluntary contraction (MVC). The method allows a convenient and non-invasive collection of a large size of SMUP pool with great representativeness. It provides a useful tool for estimating the motor unit number of proximal muscles and avoids the use of intramuscular electrodes or multiple electrical stimuli which is required in currently available MUNE techniques. This method, however, does not use the same steps as the present invention in order to decompose the compound muscle action potentials.

Gazzoni et al. (2004; D01:10.1016/j.jneumeth.2004.01.002) proposed a method for the extraction and classification of single MUAPs from surface EMG: MUAPs are first identified and extracted from the interference signal (segmentation step) and then clustered in MUs using a multichannel neural network, which differs from the present invention.

Ning et al. (2015; doi: 10.1109/JBH1.2014.2328497) describe combining the K-mean clustering (KMC) method and a modified convolution kernel compensation (CKC) method for decomposition of multi-channel surface electromyogram (EMG) acquired in voluntary contractions and does not describe the decomposition of the compound muscle action potentials nor applying calculated filters of individual motor units or a common filter of several motor units to input EMG signals captured during stimulated muscle contractions to decompose CMAPs into contributions of individual motor units. The present invention differs from this method in several important steps.

Holobar et al. (2007; DOI: 10.1109/TSP.2007.896108) disclose a decomposition technique, suitable for blind separation of linear mixtures of signals comprising finite-length symbols. The observed symbols are first modelled as channel responses in a multiple-input-multiple-output (MIMO) model, while the channel inputs are conceptually considered sparse positive pulse trains carrying the information about the symbol arising times. Described decomposition approach compensates channel responses and aims at reconstructing the input pulse trains directly. The algorithm is derived first for the overdetermined noiseless MIMO case. A generalized scheme is then provided for the underdetermined mixtures in noisy environments. Although blind, the proposed technique approaches Bayesian optimal linear minimum mean square error estimator and is, hence, significantly noise resistant. This method can be applied to EMG signals acquired in voluntary contractions and does not describe the decomposition of the compound muscle action potentials nor applying calculated filters of individual motor units or a common filter of several motor units to input EMG signals captured during stimulated muscle contractions to decompose CMAPs into contributions of individual motor units. The present invention differs from this method in several important steps.

Description of the solution of the technical problem

The technical problem of decomposing the compound muscle action potentials, CMAPs, into contributions of individual motor units is solved by a computer-implemented or circuit-implemented method comprising the following steps:

> f) receiving multichannel electromyograms, i.e. EMGs captured during voluntary muscle contractions, as input signals,
> g) identifying firing moments of motor units from EMG input signals captured during voluntary muscle contractions,
> h) calculating filters of individual motor units or a common filter of several motor units,
> i) receiving multichannel EMGs captured during stimulated muscle contractions, as input signals, and
> j) applying calculated filters of individual motor units or a common filter of several motor units to input EMG signals captured during stimulated muscle contractions to decompose CMAPs into contributions of individual motor units.

The invention will be further described in more detail based on figures, which show:

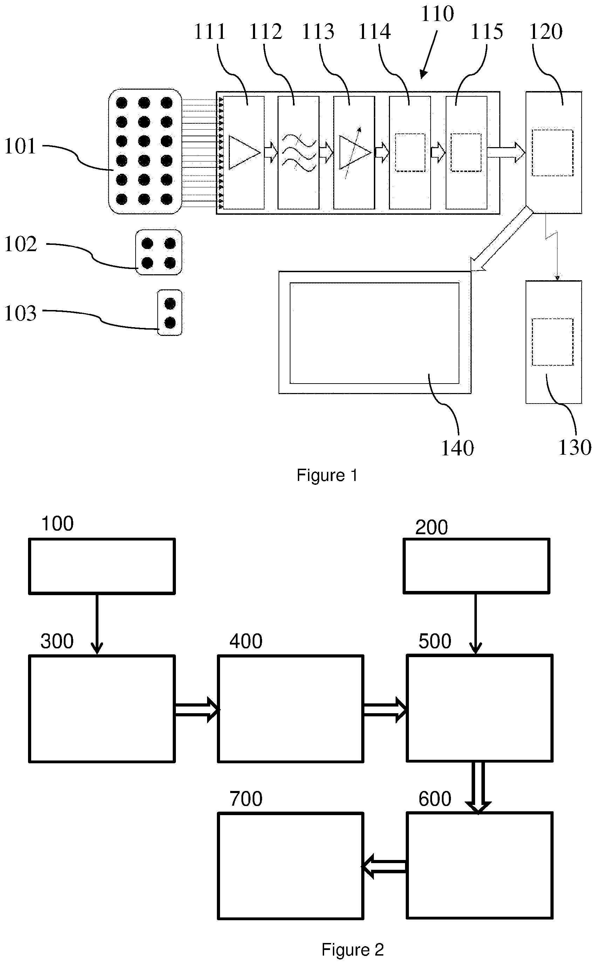

> Figure 1: Device for decomposition of compound muscle action potentials
> Figure 2: Method for decomposition of compound muscle action potentials

The technical problem of decomposition of compound muscle action potentials, CMAPs, into contributions of individual motor units is solved by the computer- or circuit-implemented method comprising the steps:

> f) receiving 100 multichannel electromyograms, i.e. EMGs captured during voluntary muscle contractions, as input signals,
> g) identifying 300 firing moments of motor units from EMG input signals captured during voluntary muscle contractions,
> h) calculating 400 filters of individual motor units or a common filter of several motor units,
> i) receiving 200 multichannel EMGs captured during stimulated muscle contractions, as input signals, and
> j) applying 500 calculated filters of individual motor units or a common filter of several motor units to input EMG signals captured during stimulated muscle contractions to decompose CMAPs into contributions of individual motor units.

The input data in step f) are provided in the form of an $M \times N_1$ matrix of EMG channel samples recorded during voluntary muscle contractions. Its $n^{th}$ column indicates the $n^{th}$ samples of all the acquired EMG channels:

$$y(n) = [y_1(n), y_2(n) \ldots y_M(n)]^T,$$

where n =1,2... $N_1$ and $y_m(n)$ is the $m^{th}$ channel of EMG signals.

In step g), known methods for the decomposition of EMG signals during voluntary muscle contractions are used to identify the P firing moments of the $j^{th}$ motor unit. These firing moments are marked with $n_{j,p}; p = 1,2 ... P$. The process of identifying firing moments is repeated for j = 1, 2... J motor units.

In step h), a block of EMG samples acquired during voluntary muscle contractions, from which the firing moments of motor units were identified in step g) is extended by adding F of differently delayed repetitions of each EMG channel:

$$\bar{y}(n) = [y_1(n), y_1(n-1) ... y_1(n-F-1) ... y_M(n) ... y_M(n-F-1)]^T$$

The correlation matrix $C_{\bar{y}}$ is then calculated:

$$C_{\bar{y}} = \left(\sum_k \bar{y}(k)\bar{y}(k)^T\right)/N_1$$

The inverse or pseudoinverse of the matrix $C_{\bar{y}}$ is calculated and the filter of the $j^{th}$ motor unit is calculated:

$$F_j = \sum_{p=1}^{P} \bar{y}(n_p)^T$$

The number P of the motor unit firings is considered an arbitrarily adjustable parameter that allows setting the decomposition accuracy, efficiency and speed. The larger the P, the more accurate and the slower the decomposition.

The input data in step i) are provided in the form of an M $\times$ $N_2$ matrix of EMG channel samples recorded during stimulated muscle contractions, which also includes a CMAP. Its $n^{th}$ column indicates the $n^{th}$ samples of all acquired EMG channels:

$$z(n) = [z_1(n), z_2(n) ... z_M(n)]^T,$$

where $z_m(n)$ is the $m^{th}$ channel of EMG signals.

Then, in step j), a block of EMG samples is extended by adding F of differently delayed repetitions of each EMG channel:

$$\bar{z}(n) = [z_1(n), z_1(n-1) ... z_1(n-F-1) ... z_M(n) ... z_M(n-F-1)]^T,$$

where F is 0 or any natural number.

The correlation matrix value $C_{\bar{z}}$ is then calculated:

$$C_{\bar{z}} = \left(\sum_k \bar{z}(k)\bar{z}(k)^T\right)/N_2$$

The matrix inverse or pseudoinverse of the matrix $C_{\bar{z}}$ is then calculated. Finally, the firing moments of the $j^{th}$ motor unit in the CMAP are estimated:

$$t_j(n) = F_j\left(C_{\bar{y}} + C_{\bar{z}}\right)^{-1}\bar{z}(n)$$

Convulsively, extended multichannel EMG signals in steps h) and j) can be represented by the following convolutional data model:

$$\bar{y}(n) = Ht(n) + \bar{\omega}(n)$$

$$\bar{z}(k) = \mathrm{Ht}(k) + \bar{\omega}(k),$$

where $\bar{\omega}(n) = [\omega_1(n) \ldots \omega_M(n - F - 1)]^T$ is an extended vector of the $n^{th}$ noise patterns.

Mixing matrix

$$\mathrm{H} = \begin{bmatrix} \mathrm{H}_1 \ldots \mathrm{H}_J \end{bmatrix}$$

with

$$\mathrm{H}_j = \begin{bmatrix} H_{1j} \\ \vdots \\ H_{Mj} \end{bmatrix}, \qquad j=1,\ldots,\mathrm{N}$$

combines the block of the $K$ MUAP samples of the $i^{th}$ motor unit as detected by the $j^{th}$ channel of the EMG signals.

$$\mathrm{H}_{ij} = \begin{bmatrix} h_{ij}(1) & \ldots & h_{ij}(K) & \ldots & 0 \\ \vdots & \ddots & \ddots & \ddots & \vdots \\ 0 & \ldots & h_{ij}(1) & \ldots & h_{ij}(K) \end{bmatrix},$$

where $h_{ij}(l)$ is the $l^{th}$ MUAP sample.

Extended vector of motor unit firing trains, also known as spike trains

$$\bar{t}(n) = \begin{bmatrix} t_1(n) \ldots t_1(n - K - F + 1) \ldots t_J(n - K - F + 1) \end{bmatrix}^T$$

contains a block $K+F$ of concurrent samples of firing trains of all active motor units. In this case, the firing train of the $j^{th}$ motor unit is represented as

$$t_j(n) = \sum_k \delta\left(n - \tau_j(k)\right)$$

where $\delta(.)$ is the Dirac impulse and the $k^{th}$ firing of the $j^{th}$ motor unit appears in time $\tau_j(k)$.

The device of the invention calculates the firing trains, and thus firing moments, of the $j^{th}$ motor unit in the CMAP as

$$\widehat{t_j}(n) = F_j\left(\mathrm{C}_{\bar{y}} + \mathrm{C}_{\bar{z}}\right)^{-1} \bar{z}(n)$$

$$\approx \mathrm{C}_{t_j\bar{t}}\mathrm{H}^T(2 \cdot \mathrm{HC}_{\bar{t}}\mathrm{H}^T + 2 \cdot \mathrm{C}_{\bar{\omega}})^{-1}\mathrm{H}\bar{t}(k) + o(n)$$

$$\approx \mathrm{C}_{t_j\bar{t}}(2 \cdot \mathrm{C}_{\bar{t}})^{-1}\bar{t}(k) = 2 \cdot t_j(n) + o(n)$$

where $o(n)$ combines the influence of noise and potentially incomplete cancellation of the mixing matrix H. The influence of $(n)$ in the estimate of $\widehat{t_j}(n)$ can be cancelled by means of arbitrary segmentation procedures, which in $\widehat{t_j}(n)$ recognise the impulses, i.e. spikes, and thus the firing moments of the motor unit.

Thus, the process according to the invention cancels the effects of MUAPs and directly estimates the firing moments of the $j^{th}$ motor unit in the CMAP from the EMG signals. The firing moments of motor units allow us to identify the delay (latency) of the motor unit response to the stimulus, its recruitment threshold and the frequency of firing during voluntary and stimulated muscle contractions. The motor unit action potentials can be used, *inter alia,* to assess the speed of conduction of action potentials along the muscle fibre and the location of the innervation zone. All the mentioned physiological parameters carry important information about the motor system, which can be assessed in further steps of the method by identifying 600 physiological properties of motor units that contribute to a CMAP, and displaying 700 the results.

The input signals received in step f) or i) are provided by a method comprising the steps of:

a) capturing EMG signals during voluntary or stimulated muscle contractions,
b) pre-amplifying the captured signals,
c) bandpass filtering the pre-amplified signals,
d) amplifying the bandpass-filtered signals,
e) converting the amplified, bandpass-filtered signals into a digital form.

The subject of the invention is also a device for decomposing CMAPs into contributions of individual motor units, comprising a processing unit 120 adapted to perform the steps of the above-described method from f) to j). The processing unit can be formed as a microprocessor or a special-purpose circuit. The device according to the invention enables the decomposition of CMAPs to the contributions of individual motor units or to any combination of contributions of individual motor units.

The device can further comprise:

- electrodes 101, 102, 103 for measuring the electrical activity of muscles, and
- an electronic circuit 110 with a high-impedance analogue input, further comprising:

  • an instrumentation amplifier 111,
  • a filter 112,
  • an amplifier 113,
  • an analogue-to-digital converter 114, and
  • a communication circuit 115.

The device obtains the CMAPs from EMG signals measured by one-dimensional or two-dimensional fields of electrodes 101, 102 or 103 with any number of invasive or surface electrodes. The electrodes are connected with the electronic circuit 110 that processes the signals to enter the processing unit 120 which performs the computer decomposition of the CMAPs using computer algorithms. The processing unit can be a stand-alone unit or integrated into an electronic circuit for capturing EMG signals.

The electrodes 101, 102, 103 are connected to a high-impedance analogue input of the electronic circuit 110 which allows the detection of multi-channel EMG signals. The instrumentation amplifier 111 amplifies each captured EMG signal channel and filters it by the bandpass filter 112. This eliminates some of the interference due to unstable contact between the skin surface and the electrodes. In the case of EMG surface signals, the frequency passband is typically set between 20 Hz and 500 Hz. In the case of needle measurements, the frequency passband is typically set between 200 Hz and 5000 Hz. Other settings are possible, too. The amplifier 113 amplifies the EMG signals, and the analogue-to-digital converter 114 converts them into a digital recording. The communication circuit 115 transmits the digital signals to the processing unit or the special-purpose circuit 120 via a wired or wireless connection. The inputs of the electronic circuit 110 must be galvanically separated from the supply voltage and outputs.

The device may further comprise a central computer 130 for further processing the decomposition results and a display 140 for displaying the decomposition results.

The subject of the invention is also a computer-readable medium having instructions stored thereon, which, when being executed by the processing unit 120, cause the described method for decomposing the compound muscle action potentials to be performed.

The subject of the invention is also a computer program comprising instructions that are executed by a computer when the program runs, and cause the computer to perform the described method for the decomposition of compound muscle action potentials.

## Claims

1. A computer-implemented or circuit-implemented process for decomposition of compound muscle action potentials (CMAP-s) into contributions of individual motor units, said process comprising the following steps:

   f) receiving (100) multichannel electromyograms, i.e. EMGs captured during voluntary muscle contractions, as input signals,

g) identifying (300) firing moments of motor units from EMG input signals captured during voluntary muscle contractions,

h) calculating (400) filters of individual motor units or a common filter of several motor units,

i) receiving (200) multichannel EMGs captured during stimulated muscle contractions, as input signals, and

j) applying (500) calculated filters of individual motor units or a common filter of several motor units to input EMG signals captured during stimulated muscle contractions to decompose CMAPs into contributions of individual motor units.

2. The process according to claim 1, **characterized in that** for ensuring input signals received in step f) or i), the process further comprises the following steps:

a) capturing EMG signals during voluntary or stimulated muscle contractions,

b) pre-amplifying the captured signals,

c) bandpass filtering the pre-amplified signals,

d) amplifying the bandpass-filtered signals,

e) converting the amplified, bandpass-filtered signals into a digital form.

3. The process according to claim 1 or 2, **characterized in that** after step j) the process further comprises the steps of:

k) identification (600) of characteristics of motor units contributing to CMAP,

l) display of results (700).

4. A computer program comprising instructions, which are executed with a computer once the program is run, and cause that the computer performs the process according to claim 1.

5. The computer program according to claim 4, wherein the instructions are stored on a computer readable medium.

6. A device for decomposition of CMAPs into contributions of individual motor units, which comprises a processing unit (120) arranged to perform the process according to claim 1.

7. The device according to claim 6, wherein the processing unit (120) is a microprocessor or a special-purpose circuit.

8. The device according to claim 6 or 7, which further comprises:

- electrodes (101, 102, 103) for measuring the electrical activity of muscles, and
- an electronic circuit (110) with a high-impedance analogue input, further comprising:

  ◦ an instrumentation amplifier (111),
  ◦ a filter (112),
  ◦ an amplifier (113),
  ◦ an analogue-to-digital converter (114), and
  ◦ a communication circuit (115).

**Patentansprüche**

1. Computerimplementierter oder schaltungsimplementierter Prozess zur Zerlegung von zusammengesetzten Muskelwirkungspotenzialen (CMAPs) in Beiträge einzelner motorischer Einheiten, wobei der Prozess folgende Schritte umfasst:

f) Empfangen (100) von Mehrkanal-Elektromyogrammen, d. h. EMGs, die während freiwilliger Muskelkontraktionen als Eingangssignale erfasst werden,

g) Identifizieren (300) von Schussmomenten motorischer Einheiten aus EMG-Eingangssignalen, die während freiwilliger Muskelkontraktionen erfasst werden,

h) Berechnen (400) von Filtern einzelner motorischer Einheiten oder eines gemeinsamen Filters mehrerer motorischer Einheiten,

i) Empfangen (200) von Mehrkanal-EMGs, die während stimulierter Muskelkontraktionen als Eingangssignale erfasst werden, und

j) Anwenden (500) berechneter Filter einzelner motorischer Einheiten oder eines gemeinsamen Filters mehrerer motorischer Einheiten auf die Eingabe von EMG-Signalen, die während stimulierter Muskelkontraktionen erfasst werden, um CMAPs in Beiträge einzelner motorischer Einheiten zu zerlegen.

2. Verfahren nach Anspruch 1, **dadurch gekennzeichnet, dass** das Verfahren zur Sicherstellung der in Schritt f) oder i) empfangenen Eingangssignale ferner die folgenden Schritte umfasst:

a) Erfassen von EMG-Signalen während freiwilliger oder stimulierter Muskelkontraktionen,
b) Vorverstärken der erfassten Signale,
c) Bandpassfiltern der vorverstärkten Signale,
d) Verstärken der bandpassgefilterten Signale,
e) Umwandeln der verstärkten, bandpassgefilterten Signale in eine digitale Form.

3. Verfahren nach Anspruch 1 oder 2, **dadurch gekennzeichnet, dass** das Verfahren nach Schritt j) ferner die folgenden Schritte umfasst:

k) Identifizieren (600) von Merkmalen motorischer Einheiten, die zu CMAP beitragen,
l) Anzeigen von Resultaten (700).

4. Computerprogramm, das Anweisungen umfasst, die mit einem Computer ausgeführt werden, sobald das Programm ausgeführt wird, und bewirken, dass der Computer den Prozess gemäß Anspruch 1 ausführt.

5. Computerprogramm nach Anspruch 4, wobei die Anweisungen auf einem computerlesbaren Medium gespeichert werden.

6. Vorrichtung zur Zerlegung von CMAPs in Beiträge einzelner motorischer Einheiten, die eine Verarbeitungseinheit (120) umfasst, die eingerichtet ist, den Prozess nach Anspruch 1 durchzuführen.

7. Vorrichtung nach Anspruch 6, wobei die Verarbeitungseinheit (120) ein Mikroprozessor oder eine Spezialschaltung ist.

8. Vorrichtung nach Anspruch 6 oder 7, ferner umfassend:

- Elektroden (101, 102, 103) zum Messen der elektrischen Aktivität von Muskeln, und
- eine elektronische Schaltung (110) mit einem hochohmigen Analogeingang, ferner umfassend:

  ◦ einen Instrumentierungsverstärker (111),
  ◦ einen Filter (112),
  ◦ einen Verstärker (113),
  ◦ einen Analog-Digital-Wandler (114), und
  ◦ eine Kommunikationsschaltung (115).

**Revendications**

1. Un procédé et dispositif de décomposition de potentiels d'action musculaire composites (PAM) en contributions des unités motrices individuelles, ledit procédé comprenant les étapes suivantes :

f) la réception (100) d'électromyogrammes multicanaux, c.-à-d. d'EMG captés lors de contractions musculaires volontaires, en tant que signaux d'entrée,
g) l'identification (300) des moments de déclenchements des unités motrices par le biais des signaux d'entrée EMG captés lors des contractions musculaires volontaires,
h) le calcul (400) des filtres des unités motrices individuelles ou d'un filtre commun de plusieurs unités motrices,
i) la réception (200) des EMG multicanaux captés lors des contractions musculaires stimulées, en tant que signaux d'entrée, et
j) l'application (500) des filtres calculés des unités motrices individuelles ou d'un filtre commun de plusieurs unités motrices aux signaux EMG d'entrée captés lors des contractions musculaires stimulées pour décomposer les PAM en contributions des unités motrices individuelles.

2. Le procédé selon la revendication 1, **caractérisé en ce que** pour garantir les signaux d'entrée captés lors de l'étape f) ou i), le procédé comprend en outre les étapes suivantes :

a) le captage des signaux EMG pendant les contractions musculaires volontaires ou stimulées,
b) la préamplification des signaux captés,
c) le filtrage passe-bande des signaux préamplifiés,
d) l'amplification des signaux soumis au filtrage passe-bande,
e) la conversion des signaux amplifiés soumis au filtrage passe-bande en format numérique.

3. Le procédé selon la revendication 1 ou 2, **caractérisé en ce que** après l'étape j), le procédé comprend en outre les étapes de :

k) l'identification (600) des caractéristiques des unités motrices contribuant aux PAM,
l) l'affichage des résultats (700).

4. Un programme informatique comprenant des instructions qui sont exécutées par un ordinateur une fois que le programme a été lancé, et qui font en sorte que l'ordinateur réalise le procédé selon la revendication 1.

5. Le programme informatique selon la revendication 4, où les instructions sont stockées sur un support lisible par ordinateur.

6. Un dispositif pour la décomposition des PAM en contributions des unités motrices individuelles, qui comprend une unité de traitement (120) réglée pour effectuer le procédé selon la revendication 1.

7. Le dispositif selon la revendication 6, où l'unité de traitement (120) est un microprocesseur ou un circuit à usage spécifique.

8. Le dispositif selon les revendications 6 ou 7, qui comprend également :

- des électrodes (101, 102, 103) pour la mesure de l'activité électrique des muscles, et
- un circuit électronique (110) doté d'une entrée analogique de haute impédance, comprenant également :

◦ un amplificateur de mesure (111),
◦ un filtre (112),
◦ un amplificateur (113),
◦ un convertisseur analogique-numérique (114), et
◦ un circuit de communication (115).

Figure 1

Figure 2